# EUROPEAN PATENT APPLICATION

(11) **EP 1 621 544 A1**
(43) Date of publication of application: **01.02.2006**
(21) Application number: 04721333.5
(22) Date of filing: 17.03.2004
(51) Int. Cl.: C07H 17/08, A61K 31/7048, A61P 9/00, A61P 9/10, A61P 43/00

(54) **REMEDIES FOR DISEASES CAUSED BY STRENGTHENED VASCULAR SMOOTH MUSCLE USING 14-MEMBERED RING MACROLIDE COMPOUNDS**

(30) Priority: 24.04.2003 JP 2003120068
(71) Applicant: Japan Science and Technology Agency, Kawaguchi-shi, Saitama 332-0012 (JP)
(72) Inventor: OSANAI, Tomohiro, Hirosaki-shi, Aomori 036-8171 (JP)
(74) Representative: Strehl Schübel-Hopf & Partner
(86) International application number: PCT/JP2004/003598
(87) International publication number: WO 2004/094446

(57) **Abstract**

It is intended in this invention to provide novel preventives/remedies for diseases caused by the growth of vascular smooth muscles, for example, re-occlusion occurring after a ballon-dilation treatment for cardiac coronary occlusion. Namely, vascular smooth muscle growth inhibitors, cyclin-dependent kinase complex expression enhancers, preventives/remedies for diseases caused by the growth of vascular smooth muscles, a method for treating these diseases, etc. with the use of, as the active ingredient, 14-membered ring macrolide compounds such as erythromycin, its derivative, roxithromycin or its derivative.

## Description

### Technical Field

The present invention relates to a preventive and/or therapeutic agent (remedy) for diseases caused by the proliferation or growth of vascular smooth muscle, comprising 14-membered ring macrolide compounds as an active ingredient.

### Background of the Invention

The 14-membered ring macrolide compounds such as erythromycin and roxithromycin are antiboitocs formed by binding of 14-membered ring lactone with side chains such as a methyl chain and dimethylamino sugar or neutral sugar, which is produced by actinomyces. They have been widely used in a clinical aspect for infectious diseases caused by gram-positive bacteria, some kinds of gram-negative bacteria, mycoplasma and chlamydia due to their strong anti-bacterial activity. Their mechanism of action is to work on 50S subunit of 70S ribosome of bacteria and to inhibit the reaction of transpeptidase so as to suppress protein synthesis.

It has been recently reported that ischemic heart disease patients have a significantly higher titer of IgG antibody against Chlamydia pneumonia than healthy individuals (please refer to Non-Patent Document 1).

Furthermore, an epidemiological report has been published that infection with Chlamydia pneumonia would increase 2~4 times the frequency of occurrence of ischemic diseases (for example, Non-Patent Document 2).

Another report supporting the above epidemiological result has been also published that Chlamydia pneumonia is detected at a high ratio in an atheromatous lesion part in cardiac coronary artery of the ischemic heart disease patients (for example, Non-Patent Document 3).

On the other hand, it is known that rapamycin belonging to the macrolide compounds has an inhibiting activity of the proliferation of vascular smooth muscles, and it has been now revealed that such activity is caused by the inhibition of growth stimuli due to various cytokines (for example, Non-Patent Document 4).
[Non-Paten Document 1]
   Saikku et al., Lancet, No.2, pp983-986, 1988
[Non-Paten Document 2]
   Danish et al., Lancet, No.350, pp430-436, 1997
[Non-Paten Document 3]
   Shor et al., Surgery after Medicine Journals, No.82, p158, 1992
[Non-Paten Document 4]
   Mario et al., Z Kardiol, Supple 3, pplll/49-lll/57, 2002

Recently, obstruction (occlusion) in cardiac coronary artery is treated to be dilated with a balloon. However, it has now become a clinical problem that obstruction will occur again after such operation. It is supposed that this re-obstruction is caused by hypertrophy of the vascular smooth muscles in damaged parts, which has occurred upon stimulation by the damage in an inner membrane of vascular.

Rapamycin, however, has a strong cytotoxicity, which would cause various side effects upon its administration, reducing immune strength of animals treated with it.

At the moment, there is no effective, practical and therapeutic agent that has a direct inhibiting activity of the proliferation of vascular smooth muscles, an d can be used as a clinically effective means to prevent the hypertrophy of the smooth muscles.

### Summary of the Invention

The purpose of the present invention is therefore to solve the above problems, and to provide a novel preventive and/or therapeutic agent (remedy) for diseases caused by the proliferation or growth of vascular smooth muscles. The present inventor has devoted himself to find the fact that 14-membered ring macrolide compounds have a direct inhibiting activity of the proliferation of vascular smooth muscles, leading to the completion of the present invention.

The present invention accordingly provides the following aspects.
1. An inhibiting agent of the proliferation of vascular smooth muscles, comprising 14-membered ring macrolide compounds as an active ingredient.
2. An inhibiting agent according to the aspect 1 wherein the vascular smooth muscles are human coronary vascular smooth muscles.
3. An inhibiting agent according to the aspect 1 or 2 wherein the 14-membered ring macrolide compounds are selected from erythromaycin or its derivatives, or roxithromycin or its derivatives.
4. An inhibiting agent according to the aspect 3 wherein the 14 -membered ring macrolide compound is roxithromycin.
5. A potentiating agent of the expression of cyclin-dependent kinase complex (CDKIs-27), comprising 14-membered ring macrolide compounds as an active ingredient.
6. A potentiating agent according to the aspect 5 wherein the 14-membered ring macrolide compounds are selected from erythromaycin or its derivatives, or roxithromycin or its derivatives.
7. A potentiating agent according to the aspect 6 wherein the 14-membered ring macrolide compound is roxithromycin.
8. A preventive and/or therapeutic agent for diseases caused by the proliferation or growth of vascular smooth muscles, comprising 14-membered ring macrolide compounds as an active ingredient.
9. A preventive and/or therapeutic agent according to the aspect 8 wherein the disease caused by the proliferation or growth of vascular smooth muscles is arteriosclerosis or chronic vascular sclerosis concurrent with the proliferation or growth of vascular smooth muscles.
10. A preventive and/or therapeutic agent according to the aspect 8 wherein the disease caused by the proliferation or growth of vascular smooth muscles is cerebrovascular stenosis, renovascular stenosis, or myocardial infarction.
11. A preventive and/or therapeutic agent according to one of the aspects 8~10 wherein the 14-membered ring macrolide compounds are selected from erythromaycin or its derivatives, or roxithromycin or its derivatives.
12. A preventive and/or therapeutic agent according to the aspect 11 wherein the 14-membered ring macrolide compound is roxithromycin.
13. A method for the inhibition of the proliferation or growth of vascular smooth muscles, comprising administrating a therapeutically effective amount of 14-membered ring macrolide compounds.
14. A method according to the aspect 13 wherein a stage from G1 phase to S phase in a cell cycle is significantly inhibited.
15. A method according to the aspect 14 wherein the in hibition of the stage from G1 phase to S phase in a cell cycle is caused by inhibition of the production of phosphorylated retinoblastoma gene products.
16. A method according to the aspect 14 or 15 wherein the inhibition of the stage from G1 phase to S phase in a cell cycle is caused by potentiation of the expression of cyclin-dependent kinase complex (CDKIs-p27).
17. A method for the treatment of diseases caused by the proliferation or growth of vascular smooth muscles, comprising administrating a therapeutic ally effective amount of the 14-membered ring macrolide compounds.
18. A method for the prevention of re-obstruction after the operation of obstruction in cardiac coronary artery, comprising administrating a preventively effective amount of the 14-membered ring macrolide compounds.
19. A method according to one of the aspects 13~18 wherein the administration is done orally.
20. A method according to one of the aspects 13~19 wherein the 14-membered ring macrolide compounds are selected from erythromaycin or its derivative s, or roxithromycin or its derivatives.
21. A method according to the aspect 20 wherein the 14-membered ring macrolide compound is roxithromycin.

### Brief explanation of the drawings

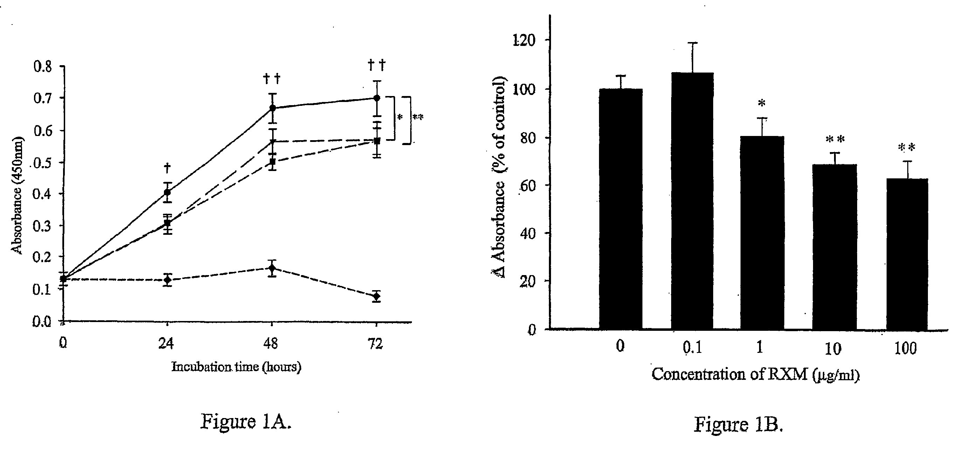

Fig.1 shows a direct inhibiting effect of RXM (roxithromycin) for the proliferation of human coronary vascular smooth muscle cells (CASMCs). "•" means a complete medium having a composition as described in Example 1, "▼" means the complete medium + RXM (1 µg/ml), "■" means the complete medium + RXM (10 µg/ml), and "◆" means serum free medium wherein fetal serum (an essential nutrient) has been removed from the complete medium. A vertical axis indicates Absorbance at 450 nm showing the cell density, and a horizontal axis indicates incubation time in Figure 1A, and concentration of RXM (µg/ml) in Figure 1 B, respectively.

Fgi.2 shows a direct inhibiting effect of general antifungal or antibacterial agent for the proliferation of CASMCs. "●" means a complete medium having a composition as described in Example 1, "▼" means the complete medium + gentamicin + Amphotericin B, "■" means the complete medium + ABPC (5 µg/ml), and "◆" means the complete medium + ABPC (50 µg/ml), and "▲" means serum free medium wherein fetal serum (an essential nutrient) has been removed from the complete medium, respectively. A vertical axis indicates Absorbance at 450 nm showing the cell density, and a horizontal axis indicates incubation time.

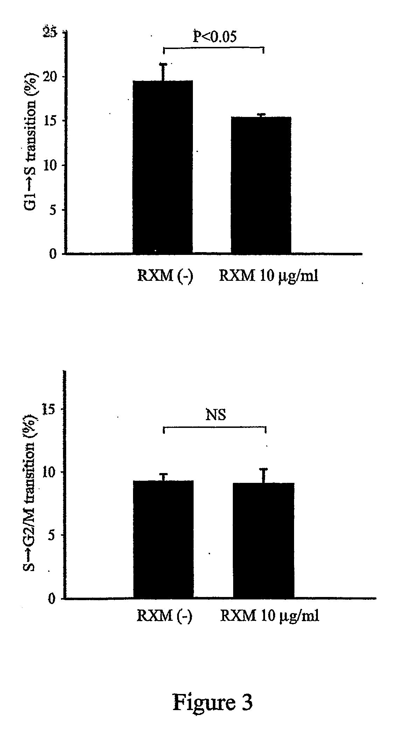

Fig.3 shows the results of analysis of cell cycle using a flow cytometry. An upper figure shows a ratio of transition from G1 phase to S phase, and a lower figure shows a ratio of transition from S phase to G2/M phase. A vertical axis indicates the ratio of transition (%), and a horizontal axis indicates the presence or non-presence of RXM (10 µg/ml).

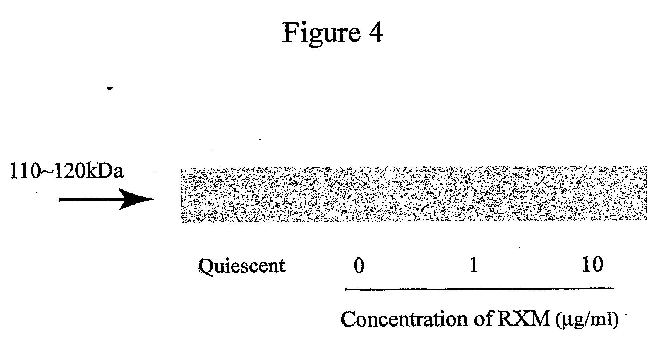

Fig.4 is a photograph obtained in an electrophoresis showing the results of analysis of phosphorylated Rb (Retinoblastoma gene products) protein by Western blot. The figures on the left side of the photo mean a molecular weight (K Dalton) of the phosphorylated Rb. "Quiescent" under the photo shows a lane of unphosphorylated (inactivated) Rb molecular, and the neighboring figures show concentration of RXM.

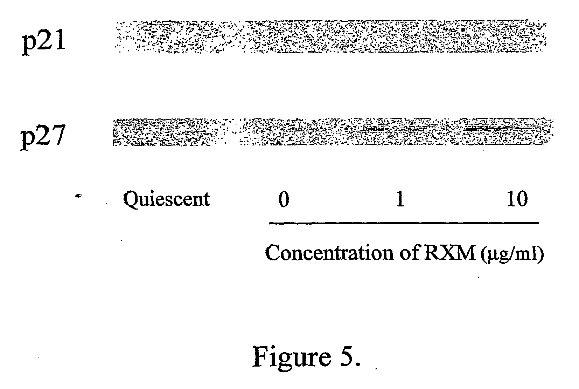

Fig.5 a photograph obtained in an electrophoresis showing the results of analysis of CDKIs proteins (p21 and p27) protein by Western blot. The figures on the left side of the photo mean a molecular weight (K Dalton) of the CDKIs proteins. The upper and lower photos show the results of CDKIs-p21 and CDKIs-p27, respectively. "Quiescent" under the photos shows a lane of (inactivated) CDKIs proteins obtained from inactivated cells without stimulation by mitogen, and the neighboring figures show concentration of RXM.

### Best mode for carrying out the invention

According to a preferable aspect of the present inhibiting agent of the proliferation of vascular smooth muscles, comprising 14-membered ring macrolide compounds as an active ingredient, it is characterized to inhibit the stage from G1 phase (preparation of DNA synthesis) to S phase (DNA synthesis) in a cell cycle of the vascular smooth muscles.

The vascular smooth muscles are found at a blood vessel wall, and are unvoluntary muscles responsible for maintainance of tension and contraction. There is no paticular limitation on their origins and parts in animal species, coronary vascular smooth muscles and main arterial vascular smooth muscles being listed as their representatives. The inhibiting agent of the proliferation according to the present invention is particularly effective for the inhibition of the proliferation of human coronary vascular smooth muscles, and is therefore effective in the prevention of re-obstruction after the operation of obstruction in cardiac coronary artery.

As shown by the examples in the present specification, the prevention of the stage from G1 phase to S phase in the cell cycle of the vascular smooth muscles is caused by the potentiation of the expression of CDKIs-p27, a major molecule in the cyclin-dependent kinase complex playing a major role in the cell cycle of eucaryotic organisms. The present invention is therefore related to the potentiating agent of the expression of cyclin-dependent kinase complex (CDKls-p27), comprising 14-membered ring macrolide compounds as an active ingredient.

The present invention further relates to the preventive and/or therapeutic agent for diseases caused by the proliferation or growth of vascular smooth muscles, comprising 14-membered ring macrolide compounds as an active ingredient. Representative examples of such diseases are arteriosclerosis or chronic vascular sclerosis concurrent with the proliferation or growth of vascular smooth muscles, cerebrovascular stenosis, renovascular stenosis, or myocardial infarction.

Furthermore, the present invention relates to the method for the inhibition of the proliferation or growth of vascular smooth muscles, comprising administrating a therapeutically effective amount of 14-membered ring macrolide compounds. As shown in the examples of the present specification, the inhibition of the proliferation or growth of vascular smooth muscles is caused by the inhibition of production of phosphorylated retinoblastoma gene products, which, in turn, is caused by the potentiation of the expression of cyclin-dependent kinase complex (CDKIs-p27).

The present invention further relates to the method for the treatment of diseases caused by the proliferation or growth of vascular smooth muscles, comprising administrating a therapeutically effective amount of 14-membered ring macrolide compounds, and to the method for the prevention of re-obstruction after the operation of obstruction in cardiac coronary artery, comprising administrating a preventively effective amount of 14-membered ring macrolide compounds.

There is no limitation on the kind of the 14-membered ring macrolide compounds that are used as the active ingredient in the present invention, and any 14-membered ring macrolide compound known for those skilled in the art may be used in the present invention.

Preferred 14-membered ring macrolide compounds are erythromaycin represented by the following formula (I), roxithromycin represented by the following formula (II), or their derivatives.

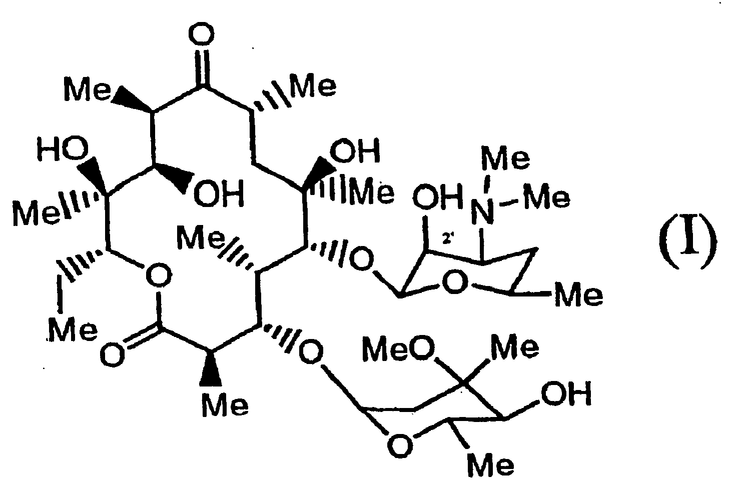

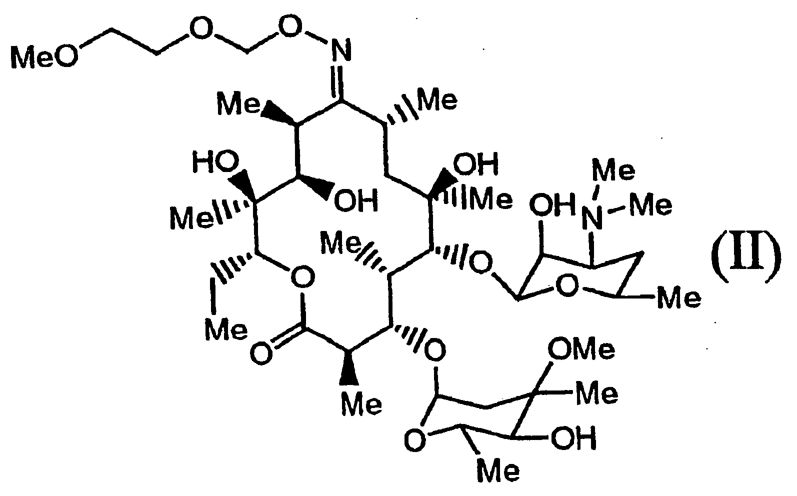

The 14-membered ring macrolide compounds are known and easily available as an agent or industrial material. Acute toxicity of erythromaycin hydrochloride (mouse LD50) is 425.6 ± 15.7 mg/kg (vein) and 490 ± 30.4 mg/kg (interperitoreal).

The 14-membered ring macrolide compounds used in the present invention may be formed into salts or esters. Preferred examples of their salts are those with inorganic acids, organic acids, inorganic bases, organic bases, acidic or basic amino acids and the like. The salts may be formed by the acids or bases at an appropriate ratio of 0.1~5 molecules per one molecule of the compound.

Preferred salts with inorganic acids are those with hydrochloride, hydrobromide, sulfuric acid, nitric acid, phosphoric acid and the like. Preferred salts with organic acids are those with acetic acid, succinic acid, fumaric acid, maleic acid, tartaric acid, citric acid, lactic acid, stearic acid, benzoic acid, methansulfonic acid, p-toluenesulfonic acid and the like.

Preferred salts with inorganic bases are those with alkaline metals such as potassium and sodium; alkaline earth metals such as calcium and magnesium; aluminum; ammonium and the like. Preferred salts with organic bases are those with diethylamine, diethanolamine, meglumine, N,N'-dibenzylethylenediamine and the like.

Preferred salts with acidic amino acids are those with those with asparatic acid, glutamic acid and the like, and preferred salts with basic amino acids are those with arginine, lysine, ornithine and the like.

Examples of esters of the present compounds are 2'-acetylester, the ester with various fatty acids and the like.

The derivatives of the 14-membered ring macrolide compounds mean any derivatives known for those skilled in the art, including various erythromycin derivatives.

The administration route of the present compounds when used as the preventive and/or therapeutic agent is not particularly limited, including oral or parenteral (for example, intramuscular, intravenous, subcutaneous, interperitoneal, percutaneous, and mucosal such as intranasal or inhalation administration).

An effective dosage of the active ingredient in the present method may be optionally determined considering totally severity of diseases, age, sex, weight, difference in susceptibility of patients; method, timing and dose interval of administration; properties of the agent; formulation and the like. The dosage is not particularly limited, being usually about 0.1 - 2,000 mg, preferably about 1 - 1,000 mg, more preferably about 10 ~ 500 mg per day and adult, and is usually administered separately one ~ four times per day. A total dosage less than 0.1 mg per day could not effect the preventive and/or therapeutic advantage, and a total dosage more than 2,000mg per day would increase concentration of the compound in blood so as to cause eruption and the like.

The agent of the present invention may be prepared according to any method known to those skilled in the art. Formulation of the agent may be optionally selected depending on administration route and the like. For example, the agent for the oral administration may be formulated into a tablet, capsule, fine particle, powder, granule, intraorally disintegrating agent, liquid, syrup and the like. The agent for the parenteral administration may be formulated into injectable solution, drops, suppository, respiratory tonic, percutaneous absorbent, transmucosal absorbent, nasal drops, otic drops and the like. Those agents may optionally contain various auxiliary substances that are usually used in the art, depending on the kind of the formulation and the like. The agent may usually comprise the active ingredient in an amount of 1~10 % by weight of the total agent.

The present invention will be further illustrated with reference to the following examples, which will show that the 14-membered ring macrolide compounds have the inhibiting activity of the proliferation of vascular smooth muscles by way of culture experiment of human coronary vascular smooth muscles cell (CASMCs), inhibition experiment of the proliferation of CASMCs, cell cycle analysis, and Western blot analysis. However, the scope of the present invention should not be limited by these examples.

### Example 1: Culture experiment of CASMCs

The CASMCs were purchased from Clonetics Co., and cultured at 37°C, 5% CO₂ using an attached culture kit (SmGM-2) in a culture medium comprising 5% fetal bovine serum (FBS), 2 ng/ml human basic fibroblast growth factor (b -FGF), 5 µg/ml bovine insulin, and 0.5 ng/ml human epidermal growth factor.

### Example 2: Inhibition experiment of the proliferation of CASMCs

The CASMCs were cultured in a 96-well plate as in the Example 1 so that about 70∼80 % of the area of each well was occupied by the cells, then put into a nutrition-depletion state for 24 hours under a non-serum condition. Roxythromycin (RXM) was then added as a test compound to the culture medium to give various concentration (0.1, 1.0, 10, 100 µg/ml) and the cells were cultured further for 24∼72 hours. The degree of growth of the cells was assayed at each elapsed time by formazan-formation coloring test with WST-1 agent using Takara Premix WST-1 Cell Proliferation Assay System Kit. Actually, Premix WST-1 was added 10 µl each followed by detection of absorbance at 450 nm by means of a plate reader.

As shown in Figure 1A, RXM of both 1 µg/ml and 10 µg/ml inhibited the proliferation of CASMCs to a statistically significant level at any time during the observation when compared with a negative control of RXM non-added group. As seen from Figure 1 B, RXM inhibited the proliferation of CASMCs to a statistically significant level in a drug -dependence manner when compared with the negative control of RXM non-added group at a range of 1~100 µg/ml.

### Example 3: Inhibition experiment of the proliferation of CASMCs using other anti-bacterial and anti-fungal agents

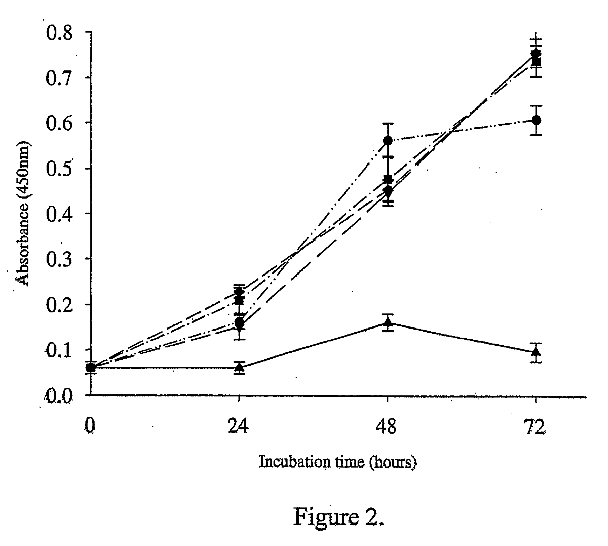

The same experiments were conducted using other anti-bacterial and anti-fungal agents. As seem Figure 2, none of the representative anti-bacterial agents, Ampicilin (ABPC; 50 µg/ml) and Gentamicin (50 µg/ml) and the representative anti-fungal agent, Amphotericin B (50 ng/ml) showed any inhibition of the proliferation of CASMCs at any time between 24 and 72 hours during the observation.

### Example 4: Cell cycle analysis

A further experiment was done using a flow cytometry in order to study when the RXM's direct inhibition activity of the proliferation of CASMCs was effected in a cell cycle.

The flow cytometry method was carried out in accordance with the following procedures and conditions.

The cells were fixed with 70% ethanol for 30 min and incubated with RNase (5 µg/ml) for 30 min. They were then incubated with propidium iodide (10 µg/ml) for 30 min, followed by detection with FACScan (Becton Dickinson Co.).

The transition ratio of the cell cycle was obtained by DNA histogram analysis with ModFit LT software, and shown as % for the total cells.

As shown in Figure 3, RXM of 10 µg/ml inhibited a stage from G1 phase to S phase in a cell cycle to a statistically significant level, while it did not inhibit a stage from S phase to G2/M phase.

### Example 5: Western blot analysis (1)

It was analyzed whether or not the RXM's inhibition of the stage from G1 phase to S phase of CASMCs was based on the inhibition of phophorylation of Rb (retinoblastoma gene products) that was supposed to be an essential intracellular molecule for transition in the cell cycle. The analysis was carried out using phosphorylated Rb as a marker by means of Western blot with a monoclonal antibody specifically recognizing phosphorylated Rb (Cell Signaling Technology Co.).

As a level of phosphorylated Rb was so low in normal conditions that it could not be detected by Western blot under normal conditions, the analysis was actually done after 5 % FBS, 2 ng/ml human b -FGF, 5 µg/ml bovine insulin, and 0.5 ng/ml human epidermal growth factor had been added to the culture medium in order to stimulate CASMCs with mitogens up to an activated state and to increase an amount of the expressed phosphorylated Rb.

Western blot was carried out as follows. After the completion of SDS gel electrophoresis, the resulting bands were transferred to PVDF membrane, blocked with dry milk, incubated successively with a first antibody (anit-phosphorylated Rb rabbit antibody) and a labeled second antibody (anti-rabbit lgG mouse antibody), developed using alkaline phosphatase immuno blot kit and determined by means of a densitometer. The operating conditions in these steps were standard ones known to those skilled in the art.

The results in Figure 4 show that RXM at aconcentration of 1~10 µg/ml clearly inhibited the production of phosphorylated Rb in CASMCs.

### Example 6: Western blot analysis (2)

In order to examine what kind of modification of intracellular molecules was responsible for the RXM's inhibition of the production of phosphorylated Rb in CASMCs, the analysis was carried out by means of Western blot in the same conditions as in Example 5, focusing attention on cyclin-dependent kinase complex (CDKls) that was known to control the intracellular molecules, especially on its major molecules, CDKls-p27 and CDKls-p21.

As an expresssion level of both CDKls-p27 and CDKls-p21 was so low in a normal cell that it could not be detected by Western blot, the analysis was actually done on the cells activated after stimulation with the mitogens in the same way as in Example 5.

The results in Figure 5 show that the expression of CDKls-p27 in CASMCs under the stimulation with the mitogens was clearly increased in a RXM concentration-dependency manner in a range of 1~10 µg/ml while no clear difference was observed for the expression of CDKIs-p21.

Accordingly, the above Western blot analyses revealed that the RXM's direct inhibition activity of the proliferation of CASMCs was based on the inhibition of the transition from G1 phase to S phase in the cell cycle, which was in turn caused mainly by the potentiation of the expression of CDKIs-p27 in the cyclin-dependent kinase complex.

### Advantages of the invention

According to the present invention, based on the inhibiting activity by the 14-membered ring macrolide compounds of the proliferation of the vascular smooth muscles, the preventive and/or therapeutic agent comprising the 14-membered ring macrolide compounds as an active ingredient is provided for diseases caused by the proliferation or growth of vascular smooth muscles, such as, for example, arteriosclerosis or chronic vascular sclerosis concurrent with the proliferation or growth of vascular smooth muscles, cerebrovascular stenosis, renovascular stenosis, or myocardial infarction.

## Claims

1. An inhibiting agent of the proliferation of vascular smooth muscles, comprising 14-membered ring macrolide compounds as an active ingredient.

2. An inhibiting agent according to Claim 1 wherein the vascular smooth muscles are human coronary vascular smooth muscles.

3. An inhibiting agent according to Claim 1 or 2 wherein the 14 -membered ring macrolide compounds are selected from erythromaycin or its derivatives, or roxithromycin or its derivatives.

4. An inhibiting agent according to Claim 3 wherein the 14-membered ring macrolide compound is roxithromycin.

5. A potentiating agent of the expression of cyclin-dependent kinase complex (CDKls-27), comprising 14-membered ring macrolide compounds as an active ingredient.

6. A potentiating agent according to Claim 5 wherein the 14-membered ring macrolide compounds are selected from erythromaycin or its derivatives, or roxithromycin or its derivatives.

7. A potentiating agent according to Claim 6 wherein the 14-membered ring macrolide compound is roxithromycin.

8. A preventive and/or therapeutic agent for diseases caused by the proliferation or growth of vascular smooth muscles, comprising 14-membered ring macrolide compounds as an active ingredient.

9. A preventive and/or therapeutic agent according to Claim 8 wherein the disease caused by the proliferation or growth of vascular smooth muscles is arteriosclerosis or chronic vascular sclerosis concurrent with the proliferation or growth of vascular smooth muscles.

10. A preventive and/or therapeutic agent according to Claim 8 wherein the disease caused by the proliferation or growth of vascular smooth muscles is cerebrovascular stenosis, renovascular stenosis, or myocardial infarction.

11. A preventive and/or therapeutic agent according to one of Claims 8~10 wherein the 14-membered ring macrolide compounds are selected from erythromaycin or its derivatives, or roxithromycin or its derivatives.

12. A preventive and/or therapeutic agent according to Claim 11 wherein the 14-membered ring macrolide compound is roxithromyc in.

13. A method for the inhibition of the proliferation or growth of vascular smooth muscles, comprising administrating a therapeutically effective amount of 14-membered ring macrolide compounds.

14. A method according to Claim 13 wherein a stage from G1 phase to S phase in a cell cycle is significantly inhibited.

15. A method according to Claim 14 wherein the inhibition of the stage from G1 phase to S phase in a cell cycle is caused by inhibition of production of phosphorylated retinoblastoma gene products.

16. A method according to Claim 14 or 15 wherein the inhibition of the stage from G1 phase to S phase in a cell cycle is caused by potentiation of the expression of cyclin-dependent kinase complex (CDKIs-p27).

17. A method for the treatment of diseases caused b y the proliferation or growth of vascular smooth muscles, comprising administrating a therapeutically effective amount of the14-membered ring macrolide compounds.

18. A method for the prevention of re-obstruction after the operation of obstruction in cardiac coronary artery, comprising administrating a preventively effective amount of the 14-membered ring macrolide compounds.

19. A method according to one of Claims 13~18 wherein the administration is done orally.

20. A method according to one of Claims 13~19 wherein the 14-membered ring macrolide compounds are selected from erythromaycin or its derivatives, or roxithromycin or its derivatives.

21. A method according to Claim 20 wherein the 14-membered ring macrolide compound is roxithromycin.
